# EUROPEAN PATENT SPECIFICATION

(11) **EP 2 676 595 B1**
(45) Date of publication and mention of the grant of the patent: **03.05.2017**
(21) Application number: 12821426.9
(22) Date of filing: 29.05.2012
(51) Int. Cl.: A61B 1/00

(54) **ENDOSCOPE AUXILIARY INSTRUMENT AND ENDOSCOPE**
ENDOSKOPISCHES ZUSATZINSTRUMENT UND ENDOSKOP
INSTRUMENT ENDOSCOPIQUE AUXILIAIRE ET ENDOSCOPE

(30) Priority: 09.08.2011 JP 2011174278
(43) Date of publication of application: 25.12.2013
(73) Proprietor: Olympus Corporation, Tokyo 192-8507 (JP)
(72) Inventor: NAITO, Kimihiko, Hachioji-shi Tokyo 192-8507 (JP); UEKI, Ryo, Hachioji-shi Tokyo 192-8507 (JP)
(74) Representative: Schicker, Silvia
(86) International application number: PCT/JP2012/063796
(87) International publication number: WO 2013/021710

(56) References cited:
- EP-A1- 2 116 188
- JP-A- 10 033 467
- JP-A- H11 342 104
- JP-A- 2002 301 011
- JP-A- 2003 093 325
- JP-A- 2003 093 329
- JP-A- 2003 290 134
- JP-A- 2008 017 938
- JP-A- 2010 012 172
- US-A- 5 569 268
- US-A1- 2005 090 709

## Description

### Technical Field

The present invention relates to an endoscope auxiliary instrument and an endoscope, and more particularly to an endoscope auxiliary instrument and an endoscope having a moving member that is mounted at a distal end portion of an endoscope insertion portion.

### Background Art

Conventionally, in a case where it is difficult to secure a distance between a distal end portion of an endoscope insertion portion and an object when observing the inside of a body using an endoscope, generally a tubular endoscope hood is mounted at the distal end portion of the endoscope insertion portion and observation of the inside of the body is performed. Since use of the hood makes it possible to secure a predetermined distance between the distal end portion and the object, favorable observation can be carried out with the endoscope.

However, when the hood is provided at the distal end portion, the length of a distal end rigid portion of the endoscope insertion portion increases, and there is the problem that it becomes difficult to insert the endoscope insertion portion into a curved body cavity.

As disclosed in Japanese Patent Application Laid-Open Publication No. 2002-301011, an endoscope has been proposed that has a mechanism such that a hood protrudes/retracts at a distal end portion of an endoscope insertion portion. According to the endoscope of the aforementioned application, a hood can be protruded frontward only when required, and the hood can be retracted when not required.

Further, as disclosed in Japanese Utility Model Application Laid-Open Publication No. 55-12953, an endoscope having urging means by which a hood is resiliently urged forward has also been proposed.

However, in the case of a protrudable/retractable hood, the hood itself has to be made of a material which is rigid to some extent so as to enable the hood to protrude/retract by sliding back and forth at the distal end portion of the endoscope insertion portion. Therefore, there is such a problem that a surgeon has to carefully perform insertion operation or bending operation in order to prevent the protruded rigid hood from strongly contacting a living tissue, and as a result, endoscope examination time will be increased.

In addition, if the above-described endoscope having urging means for urging the hood forward is used, even when the hood which is rigid to some extent contacts a living tissue such as mucosa, the hood retracts due to resilience of itself, thereby capable of absorbing an impact when the hood contacts the living tissue. However, the urging means has to be provided at the distal end portion, which causes a problem that a diameter of the distal end portion of the endoscope insertion portion is increased. In addition, a space for disposing the urging means is required, which causes a problem that the length of the distal end rigid portion is increased.

Therefore, an object of the present invention is to provide an endoscope auxiliary instrument and an endoscope that can absorb an impact when a hood contacts a living tissue, without increasing an outer diameter of a distal end portion of an insertion portion of the endoscope and without increasing a length of a distal end rigid portion.

Documents JP 2002/2301011 and US 5,569,268 disclose endoscope auxiliary instruments belonging to the closest prior art.

### Disclosure of Invention

### Means for Solving the Problem

An endoscope auxiliary instrument according to one aspect of the present invention is defined in appended claim 1.

An endoscope according to one aspect of the present invention includes the endoscope auxiliary instrument according to one aspect of the present invention.

### Brief Description of the Drawings

Fig. 1 is a configuration diagram illustrating the configuration of an endoscope according to an embodiment of the present invention;
Fig. 2 is a perspective view of an endoscope distal end portion 2A to which a hood unit 21 is mounted, that illustrates a state in which a hood 31 that is a moving member is not protruding from the hood unit 21 according to the embodiment of the present invention;
Fig. 3 is a perspective view of the endoscope distal end portion 2A in a state in which the hood 31 is protruding from the hood unit 21 according to the embodiment of the present invention;
Fig. 4 is a perspective view of a wire fixing portion 24 that illustrates a state in which a wire fixing portion 24 is fixed in an opening portion 16 according to the embodiment of the present invention;
Fig. 5 is a cross-sectional view of a cross-section through the axis of a distal end rigid portion 11 along a line V-V shown in Fig. 2;
Fig. 6 is a cross-sectional view of a cross-section through the axis of the distal end rigid portion 11 along a line VI-VI shown in Fig. 2;
Fig. 7 is a side view as viewed from a distal end side of a hood cover unit 31A according to the embodiment of the present invention;
Fig. 8 is a front view of the hood cover unit 31A according to the embodiment of the present invention;
Fig. 9 is a cross-sectional view as viewed from an arrow A4 direction along a line IX-IX shown in Fig. 8;
Fig. 10 is a cross-sectional view of a cross-section through the axis of the endoscope distal end portion 2A along a line VII-VII shown in Fig. 3;
Fig. 11 is a cross-sectional view of the wire fixing portion 24 in a state in which the wire fixing portion 24 is attached to the opening portion 16 according to the embodiment of the present invention;
Fig. 12 is a schematic view for describing operations of an endoscope auxiliary instrument according to the embodiment of the present invention;
Fig. 13 is a perspective view of the distal end portion 2A in a case where a colored ring is provided in an auxiliary instrument insertion port 51a according to Modification 1 of the embodiment of the present invention;
Fig. 14 is a partial cross-sectional view of the distal end portion 2A at a region including a colored ring 94, along a line XIV-XIV shown in Fig. 13;
Fig. 15 is a partial cross-sectional view of the distal end portion 2A in which the periphery of an opening portion 51a is made luminous according to Modification 2 of the embodiment of the present invention; and
Fig. 16 is a view for describing the configuration of Modification 3 of the embodiment of the present invention.

### Best Mode for Carrying Out the Invention

An embodiment of the present invention is described hereunder with reference to the drawings.

Note that the components in the drawings used in the following description are each displayed in a different contraction scale so as to be shown in a size that is recognizable in the drawings. Further, the present invention is not limited to only the quantity of components, the shapes of components, the ratios between the sizes of components, and the relative positional relationship between the respective components described in the drawings.

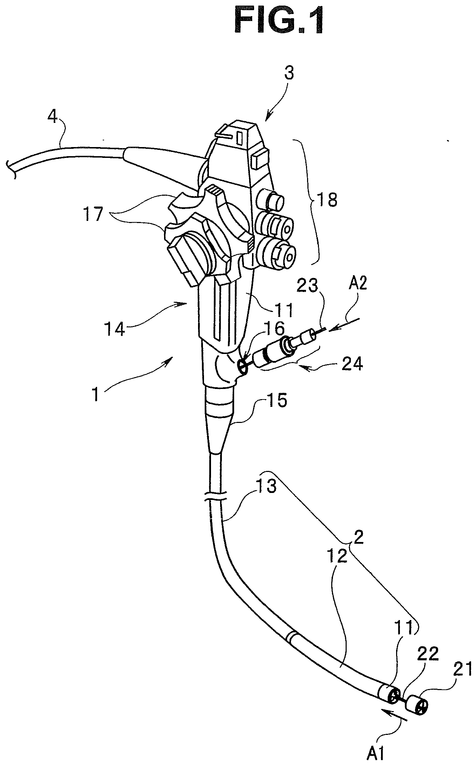

First, the configuration of an endoscope according to the present embodiment is described based on Fig. 1. Fig. 1 is a configuration diagram that illustrates the configuration of the endoscope according to the present embodiment.

An endoscope 1 includes an elongated insertion portion 2 that is inserted into a site to be observed, an operation portion 3 that is connected to a proximal end portion of the insertion portion 2, and a universal cable 4 that is extended from a side of the operation portion 3.

The elongated insertion portion 2 has a distal end rigid portion 11 at a distal end side thereof. A bending portion 12 as a bendable moving portion is connected to a proximal end side of the distal end rigid portion 11. A flexible tube portion 13 is connected to a proximal end side of the bending portion 12. The flexible tube portion 13 is long and has flexibility, and the surface thereof is formed by a flexible tubular member.

The operation portion 3 includes an operation portion body 14 that constitutes an operation/grasping portion, a bend preventing portion 15 that is connected to a proximal end side of the flexible tube portion 13 of the insertion portion 2, and an opening portion 16 of an auxiliary instrument insertion channel which is inside the insertion portion 2 that is arranged in the vicinity of the bend preventing portion 15. Although not shown in the drawings, a treatment instrument insertion port for inserting a treatment instrument is also provided in the vicinity of the bend preventing portion 15.

A plurality of bending operation knobs 17 for subjecting the bending portion 12 of the insertion portion 2 to a bending operation are rotatably arranged on the operation portion body 14, and switches 18 and the like that are operated by a user to perform air/water feeding and the like are also provided on the operation portion body 14. Note that the bending operation knobs 17 include a UD bending operation knob for subjecting the bending portion 12 to an upward/downward bending operation, and an RL bending operation knob for subjecting the bending portion 12 to a rightward/leftward bending operation, and these bending operation knobs are arranged in a superimposed manner.

A hood unit 21 is mountable to the distal end portion of the insertion portion 2. As described later, a wire cable 22 is connected to the hood unit 21. The wire cable 22 is inserted from an auxiliary instrument insertion port in the distal end portion of the insertion portion 2 and is passed through the auxiliary instrument insertion channel and is protruded from the opening portion 16, and is fixed by means of a wire fixing portion 24 that fixes the wire cable 22.

The hood unit 21, the wire cable 22, and the wire fixing portion 24 constitute an endoscope auxiliary instrument. As described later, a hood 31 that is a moving member is provided on a distal end side of the hood unit 21.

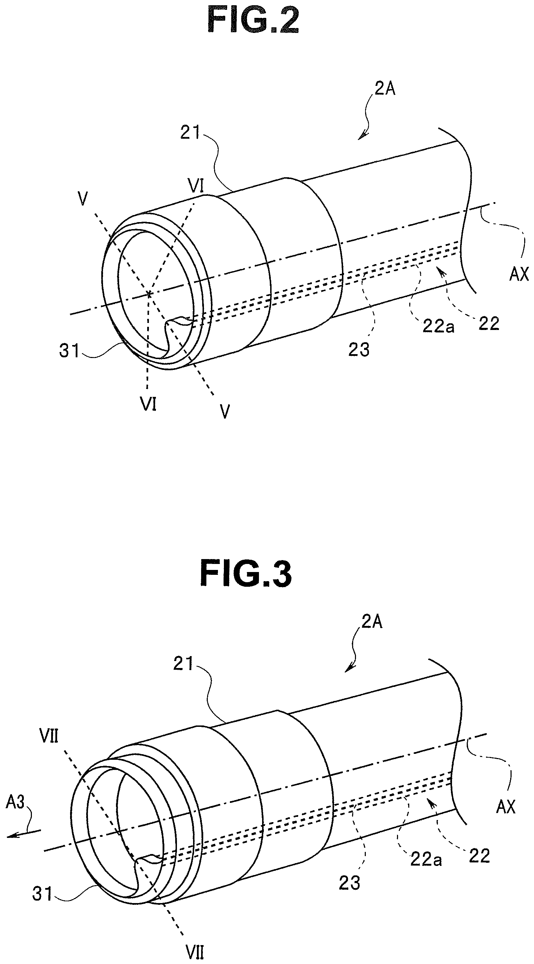

Fig. 2 is a perspective view of an endoscope distal end portion 2A in a state in which the hood unit 21 is mounted thereto and the hood 31 that is a moving member is not protruding from the hood unit 21. Fig. 3 is a perspective view of the endoscope distal end portion 2A in a state in which the hood 31 is protruding from the hood unit 21.

The hood unit 21 that is a cylindrical shape is mounted to the endoscope distal end portion 2A from the direction of an arrow A1 shown in Fig. 1. As shown in Fig. 2, the hood 31 that is capable of protruding in the distal end direction is provided on the distal end side of the hood unit 21. The hood 31 is a member that has a cylindrical shape, that is, is a cylindrical member, and as shown by an arrow A3 in Fig. 3, is configured to be capable of protruding in the distal end (frontward) direction of the endoscope distal end portion 2A along the direction of an axis AX of the endoscope distal end portion 2A.

The wire cable 22 is constituted by a tube 22a and a wire 23 that is inserted through the tube 22a, and is connected to the hood 31. The wire 23 is made of stainless steel. The tube 22a is made of a resin such as Teflon (registered trademark). As described later, when the wire 23 is pushed out towards the distal end side, the hood 31 is caused to protrude in the distal end (frontward) direction of the endoscope distal end portion 2A along the axis AX direction of the endoscope distal end portion 2A. In contrast, when the wire 23 is drawn in towards the proximal end side, the hood 31 withdraws in the proximal end (rearward) direction of the endoscope distal end portion 2A along the axis AX direction of the endoscope distal end portion 2A. Thus, the hood 31 is a moving member that is capable of protruding/retracting along the axial direction of the insertion portion 2.

The outer circumferential face of the wire 23 is covered by the tube 22a to insulate the wire 23.

One end of the wire cable 22 is fixed to the hood unit 21.

The other end of the wire cable 22 that is inserted through the inside of the auxiliary instrument insertion channel and protrudes from the opening portion 16 is fixed by the wire fixing portion 24. The wire fixing portion 24 is fixed to the opening portion 16 from a direction indicated by an arrow A2 in Fig. 1.

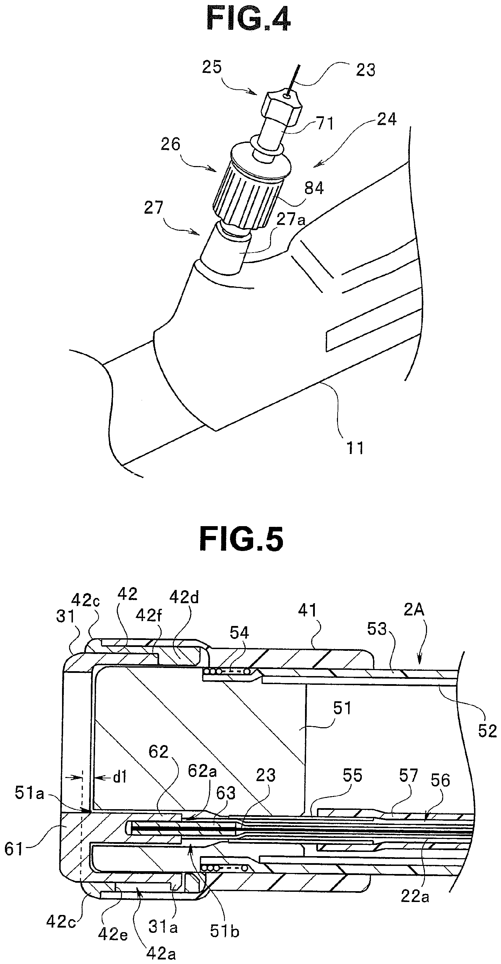

Fig. 4 is a perspective view of the wire fixing portion 24 that shows a state in which the wire fixing portion 24 is fixed to the opening portion 16. The wire fixing portion 24 includes two operation portions 25 and 26 and a connection portion 27. The operation portion 25 is an operation portion for fixing the wire cable 22. The operation portion 26 is an operation member for fixing the wire cable 22 at a desired position when the wire cable 22 has been moved in the forward/rearward direction. The connection portion 27 has a mechanism for mounting and fixing the wire fixing portion 24 in the opening portion 16.

As described later, after mounting and fixing the wire fixing portion 24 to the opening portion 16 by means of the connection portion 27, a surgeon passes the wire cable 22 through a wire insertion channel of the wire fixing portion 24. Thereafter, by means of the operation portion 25, the surgeon can fix the wire cable 22 and move the wire cable 22 back and forth in the axial direction to cause the hood 31 to protrude or retract. The protruding or retracted state can be maintained by means of the operation portion 26.

### (Configuration of hood unit)

Next, the configuration of the hood unit 21 will be described.

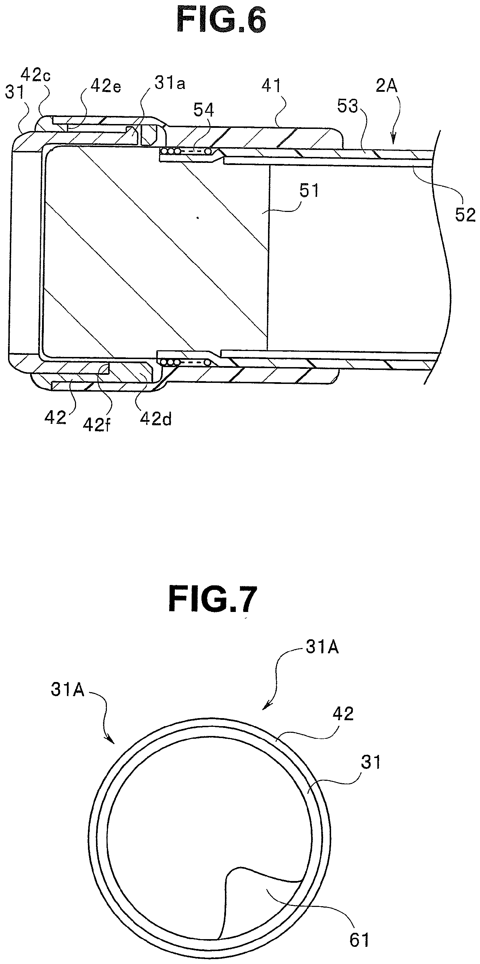

Fig. 5 is a cross-sectional view of a cross-section through an axis of the distal end rigid portion 11 along a line V-V shown in Fig. 2. Fig. 6 is a cross-sectional view of a cross-section through an axis of the distal end rigid portion 11 along a line VI-VI shown in Fig. 2.

The distal end rigid portion 11 of the endoscope insertion portion 2 includes a rigid member 51 made of stainless steel within which an image pickup device, an objective optical system, an illumination portion and the like are incorporated. A proximal end portion of the rigid member 51 is fitted into an inner diameter portion of one bending piece 52 of the bending portion 12. A distal end portion of a flexible sheath member 53 that is made of rubber that covers the bending piece 52 is fixed to the rigid member 51 by a thread winding portion 54. The thread winding portion 54 is a region around which a thread is wound and over which an adhesive is applied.

An auxiliary instrument insertion port 51a of an auxiliary instrument insertion channel is provided in a distal end face of the rigid member 51, together with an observation window, an illuminating window, and an opening portion of a treatment instrument insertion channel. In this case, the wire cable 22 of the hood unit 21 is inserted from the auxiliary instrument insertion port 51a. The hood unit 21 is mounted to the distal end portion 2A of the endoscope insertion portion 2 so as to cover the rigid member 51.

A hole 51b that connects with the auxiliary instrument insertion port 51a is formed in the rigid member 51. The wire cable 22 is inserted through the hole 51b. A pipe 55 made of stainless steel is press-fitted into a proximal end side of the hole 51b, and a channel tube 57 for forming an auxiliary instrument insertion channel 56 is connected to the proximal end side of the hole 51b so as to cover the outer circumference of a proximal end side of the pipe 55. The channel tube 57 is made of resin and is mounted in a fixed condition to the pipe 55 by means of an adhesive or the like. The wire cable 22 is inserted through the channel tube 57.

As shown in Fig. 5 and Fig. 6, the hood unit 21 includes a unit body 41 that is a body portion, the cylindrical hood 31 that is a moving member, and a hood cover 42 that is a cylindrical cover member.

The unit body 41 is a tubular member that has a step portion between a distal end side section and a proximal end side section, in which the inner diameter of the distal end side section is larger than the inner diameter of the proximal end side section. The unit body 41 is made from elastomer or the like. The hood cover 42 is inserted into the inside of a tubular portion of a distal end side section of the unit body 41 and is fixed thereto with an adhesive. The unit body 41 is configured so that the distal end portion 2A of the endoscope insertion portion 2 can be attached by being brought into intimate contact with the inside of the tubular portion of the distal end side section of the unit body 41 so as to fit thereto.

The tubular hood cover 42 is provided on the inner diameter side of the distal end side section of the unit body 41. The tubular hood 31 that is a moving member is inserted on the inner diameter side of the hood cover 42. The hood cover 42 is a holding portion that movably holds the hood 31. As described later, the hood cover 42 is a member for regulating movement of the hood 31.

The hood 31 is attached to the hood cover 42 in a manner such that the hood 31 is capable of protruding/retracting in the axial direction of the endoscope distal end portion 2A. The hood 31 and the hood cover 42 are composed of a resin such as polycarbonate. The hood 31 can be fitted into the hood cover 42 by pressing the hood 31 in from the distal end side of the hood cover 42. The fitted hood 31 is movable along the axial direction of the insertion portion 2. Hence, the hood cover 42 constitutes a holding portion that is provided at the distal end portion 2A of the insertion portion 2 of the endoscope 1, and movably holds the hood 31 along the axial direction of the insertion portion 2.

When the hood unit 21 is mounted to the distal end portion 2A of the insertion portion 2, the inner surface of the unit body 41 intimately contacts the outer circumferential face of the endoscope distal end portion 2A. The hood unit 21 is configured so that, when mounted to the distal end portion 2A of the insertion portion 2, as shown in Fig. 5, the distal end portion of the hood cover 42 protrudes further forward than the distal end face of the rigid member 51 by the amount of a distance d1. That is, the hood cover 42 that is a holding portion has a protruding portion that protrudes in the distal end direction of the distal end portion 2A more than the distal end face of the distal end portion 2A. As a result, movement of the hood 31 in a direction orthogonal to the axis of the distal end portion 2A is restricted by the protruding portion of the hood cover 42 at a position that is forward of the rigid member 51 also, and backlash when the hood 31 protrudes/retracts is prevented.

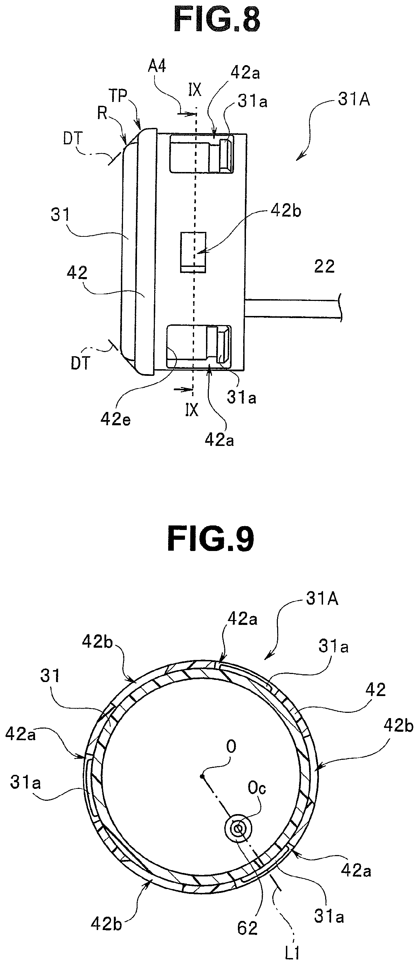

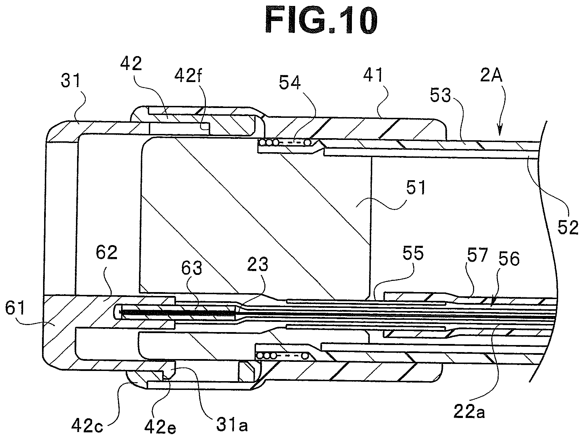

Fig. 7 to Fig. 9 are views for describing a hood cover unit 31A in which the hood 31 is inserted into the hood cover 42, and to which the wire cable 22 has been attached. Fig. 7 is a side view as viewed from the distal end side of the hood cover unit 31A. Fig. 8 is a front view of the hood cover unit 31A. Fig. 9 is a cross-sectional view as viewed from an arrow A4 direction along a line IX-IX in Fig. 8. Fig. 10 is a cross-sectional view of a cross-section through an axis of the endoscope distal end portion 2A along a line VII-VII in Fig. 3 in a state in which the hood 31 protrudes from the endoscope distal end portion 2A.

As shown in Fig. 5, the cylinder-shaped hood 31 has an inward flange portion on a distal end side. Further, as shown in Fig. 5 and Fig. 7, the hood 31 has a protruding portion 61 that protrudes to the inner diameter side at one part of the inward flange portion. The protruding portion 61 has a columnar boss 62 for fixing the distal end portion of the wire cable 22 on the inner side of a cylindrical portion of the hood 31. A hole 62a into which the distal end portion of the wire 23 in the wire cable 22 is inserted is formed in a proximal end portion of the boss 62 that is a fixing portion. The boss 62 is of a size that can enter the hole 51b of the rigid member 51.

A pipe 63 is provided in the boss 62 by insert molding. The wire 23 is inserted into the pipe 63 and fixed therein by an adhesive. The tube 22a that covers the wire 23 is mounted so as to cover an outer circumferential portion on a proximal end side of the pipe 63.

The hood 31 is a cylindrical member. Although the distal end side of the hood 31 is a cylindrical portion, the proximal end side thereof has a plurality of extending portions that extend in the proximal end direction. A claw portion 31 a is provided in each extending portion. As shown in Fig. 5 and Fig. 8, the plurality of claw portions 31a are provided so that, when the hood 31 has been inserted into the hood cover 42, the plurality of claw portions 31 a enter a plurality of rectangular holes 42a that are formed in the hood cover 42 along the axial direction.

As shown in Fig. 9, in this case three holes 42a are provided at intervals of 120 degrees around the axis of the hood cover 42. Three claw portions 31a that protrude in the outer diameter direction are provided in the hood 31 in correspondence with the three holes 42a.

Note that a plurality of holes 42b, in this case three holes, are also provided in the cylindrical shaped hood cover 42 to prevent the hood cover 42 from falling off from the unit body 41.

The hood cover 42 also has an outward flange portion 42c on the distal end side and an inward flange portion 42d on the proximal end side.

As shown in Fig. 8, the hood cover 42 has a tapered portion TP along an outer circumference of the distal end portion thereof, and the hood 31 has a curved portion R along an outer circumference of the distal end portion thereof. As indicated by an alternate long and short dash line DT in Fig. 8, the hood unit 21 is configured so that when the hood 31 has withdrawn to the proximal end side of the hood cover 42, an inclining line of the tapered portion TP of the hood cover 42 and a tangent line DT of the curved portion R of the hood 31 approximately coincide with each other in a cross section along the axial direction of the hood unit 21. As a result, smooth contact of the hood 31 with a mucous membrane or the like in the body can be realized.

As shown in Fig. 10, when the hood 31 has moved so as to protrude in the distal end direction by means of the wire cable 22, the movement of the hood 31 is stopped by the respective claw portions 31a coming in contact with an inner circumferential wall 42e on the distal end side of the corresponding hole 42a.

Further, when the hood 31 has moved so as to withdraw in the proximal end direction by means of the wire cable 22, as shown in Fig. 5 and Fig. 6, the movement of the hood 31 is stopped by a part of the proximal end face of the hood 31 coming in contact with an inner circumferential wall 42f on the proximal end side of the relevant hole 42a of the hood cover 42.

That is, the hood cover 42 that is a holding portion has movement restricting portions that restrict movement of the hood 31 along the axial direction of the insertion portion 2. Specifically, the hood 31 has the plurality of claw portions 31 a, and movement of the hood 31 along the axial direction of the insertion portion 2 is restricted by the plurality of claw portions 31a contacting against the inner circumferential wall of the respective holes 42a that are restricting portions.

Note that, as shown in Fig. 9, one of the three claw portions 31a is disposed so as to be positioned on a line L1 that, when viewed from the distal end side, passes through a central axis O of the hood cover unit 31 A and a central axis Oc of the boss 62 at which the distal end portion of the wire 23 is fixed. This is to prevent backlash when the hood 31 is protruded from the distal end portion.

### (Configuration of wire fixing portion)

Next, the configuration of the wire fixing portion 24 will be described.

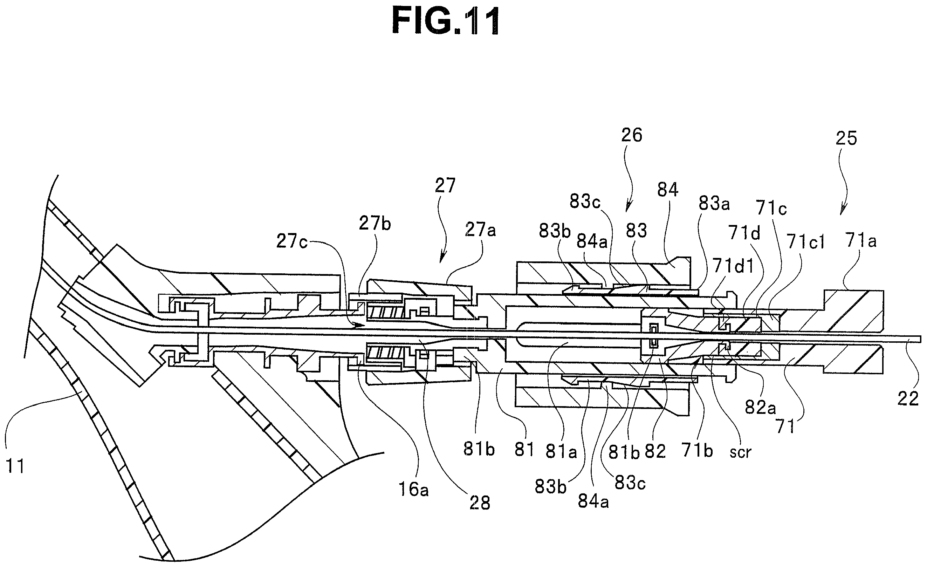

Fig. 11 is a cross-sectional view of the wire fixing portion 24 in a state in which the wire fixing portion 24 is attached to the opening portion 16. The connection portion 27 of the wire fixing portion 24 is mounted and fixed in the opening portion 16. The connection portion 27 is connected to the operation portion 26. The operation portion 25 is connected on the opposite side to the connection portion 27 of the operation portion 26.

The operation portions 25 and 26 and the connection portion 27 each have a columnar shape, and a wire insertion channel 28 through which the wire cable 22 is inserted is formed at the axial center of the of the operation portions 25 and 26 and the connection portion 27 that are connected. The wire cable 22 protrudes from the opening portion 16 and is inserted through the inside of the wire insertion channel 28.

As shown in Fig. 4 and Fig. 11, the operation portion 25 has a cylindrical-shaped handle 71 that is grasped by the surgeon and rotated around its own axis. A grasping portion 71a that has projections and depressions on an outer circumferential portion so as to facilitate operations by the surgeon is formed at the proximal end side of the handle 71.

The handle 71 is made of resin and has an opening portion 71b with a large internal diameter on an inner side on the distal end side, and a cylindrical member 71c made of stainless steel is provided on the interior side, that is, the proximal end side, inside the opening portion 71b. The cylindrical member 71c has an inward flange portion 71c1 on the proximal end side. An elastic member 71d made of silicone rubber or the like is provided on the inner side of the cylindrical member 71c. An inward flange portion 71d1 is formed on the proximal end side of the elastic member 71d. A screw portion (not illustrated in the drawings) is provided on the inner surface on the distal end side of the opening portion 71b.

The operation portion 26 includes a cylindrical body portion 81 that is made of resin, a connecting member 82 made of stainless steel that is provided on the inner side of a cylindrical portion of the body portion 81, a slider member 83 made of resin that slides in the axial direction that is provided on the outer circumference of the body portion 81, and a handle 84 that is provided on the outer circumference of the slider member 83 and rotates around its own axis.

The connecting member 82 has, on a proximal end side, a protruding portion 82a that has an outward flange portion. The inward flange portion 71d1 of the elastic member 71d and the outward flange portion of the protruding portion 82a are engaged with each other.

A screw portion (not illustrated in the drawings) is formed on the outer circumferential face on the distal end side of the connecting member 82, and the screw portion is screwed together with the screw portion on the distal end side of the handle 71 in a screwing region scr.

When the handle 71 is rotated around its own axis in a predetermined direction, the connecting member 82 moves relatively to the proximal end side with respect to the cylindrical member 71c by means of the screwing region scr. If the handle 71 is rotated further, the elastic member 71 d is compressed by the inward flange portion 71c1 and the connecting member 82, and the elastic member 71d presses the wire cable 22 from the outer circumference while intimately contacting the wire cable 22 and, as a result, the wire cable 22 is fixed with respect to the handle 71. When the handle 71 is rotated in the opposite direction, since the elastic member 71d is no longer compressed by the inward flange portion 71c1 and the connecting member 82, the wire cable 22 is not pressed by the elastic member 71d and, as a result, the wire cable 22 enters a state in which the wire cable 22 is not fixed with respect to the handle 71.

The connecting member 82 is connected with a slider member 83 by a fixing member 81b through a slit 81a that is formed in the body portion 81. Accordingly, when the connecting member 82 moves in the axial direction, the slider member 83 also moves in the same direction together therewith. Further, since the connecting member 82 connects the slider member 83 and the elastic member 71 d, when the handle 71 moves in the axial direction, the slider member 83 moves in the same direction and the wire cable 22 that is fixed to the handle member 71 also moves in the same direction.

The slider member 83 has a cylinder-shaped cylindrical portion 83a, and a plurality of extending portions 83 b that extend in the distal end direction from the cylindrical portion 83a. A screw portion (not illustrated in the drawings) is provided on one part of the outer circumferential face of the plurality of extending portions 83b of the slider member 83, and a screw portion (not illustrated in the drawings) that screws together with the screw portion of the extending portions 83b in a corresponding manner is provided on the inner surface of the handle 84.

Further, a tapered portion 83c is formed on the outer circumferential face of the respective extending portions 83b. On the inner surface of the handle 84, a ring-shaped projecting portion 84a that projects in the inner diameter direction is provided at a position that comes in contact with the tapered portion 83c of the respective extending portions 83b.

Because the screw portion of the respective extending portions 83b and the screw portion of the handle 84 are screwed together, when the handle 84 rotates around its own axis, the handle 84 moves relatively in the axial direction with respect to the slider member 83. When the handle 84 moves in the axial direction, the projecting portion 84a of the handle 84 presses the tapered portion 83a of the extending portion 83b or separates from the tapered portion 83a.

Hence, when the surgeon rotates the handle in a predetermined direction around its own axis, the handle 84 moves relatively in the proximal end side direction with respect to the slider member 83, and the projecting portion 84a presses the tapered portion 83a, and thus the slider member 83 can be fixed with respect to the body portion 81. Further, when the surgeon rotates the handle in the opposite direction to the aforementioned predetermined direction around its own axis, the handle 84 moves relatively in the distal end side direction with respect to the slider member 83 and the projecting portion 84a separates from the tapered portion 83a, and thus the slider member 83 can be made movable in the axial direction with respect to the body portion 81.

The distal end side of the body portion 81 is fixed through the fixing portion 81 b for connecting with the connection portion 27 to a proximal end portion of a connection portion body 27a of the connection portion 27. A member made of rubber is provided on the inner side of a distal end portion 27b of the connection portion body 27a, and the distal end portion 27b is detachably connected to a pipe sleeve 16a that is made of metal of the opening portion 16. The wire cable 22 is inserted from the opening portion 27c of the distal end portion 27b of the connection portion 27.

### (Operation)

The operation of the endoscope auxiliary instrument with respect to the above described configuration is as follows. The surgeon inserts the proximal end portion of the wire cable 22 that is connected to the hood unit 21 that is included in the endoscope auxiliary instrument from the auxiliary instrument insertion port 51a of the endoscope distal end portion 2A, passes the proximal end portion of the wire cable 22 through the auxiliary instrument insertion channel 56, and causes the proximal end portion of the wire cable 22 to protrude from the opening portion 16.

After mounting and fixing the wire fixing portion 24 to the opening portion 16 by means of the connection portion 27, the surgeon inserts the proximal end portion of the wire cable 22 from the opening portion 27b on the distal end side of the wire fixing portion 24, passes the proximal end portion of the wire cable 22 through the wire insertion channel 28 inside the operation portions 25 and 26 and the connection portion 27, and causes the proximal end portion of the wire cable 22 to protrude from the opening in the proximal end portion of the operation portion 25.

In a state in which the wire cable 22 is pulled and the hood 31 is retracted, that is, withdrawn, the surgeon rotates the handle 71 around its own axis to fix the wire cable 22 with respect to the handle 71. At that time, the surgeon also rotates the handle 84 around its own axis to fix the wire cable 22 with respect to the wire fixing portion 24.

When using the hood 31, the surgeon rotates the handle 84 around its own axis in the opposite direction to enter a state in which the wire cable 22 is not fixed with respect to the wire fixing portion 24, and then pushes the handle 71 to push the wire cable 22 outward in the distal end direction. As a result, the hood 31 that is connected to the distal end of the wire cable 22 protrudes from the endoscope distal end portion 2A.

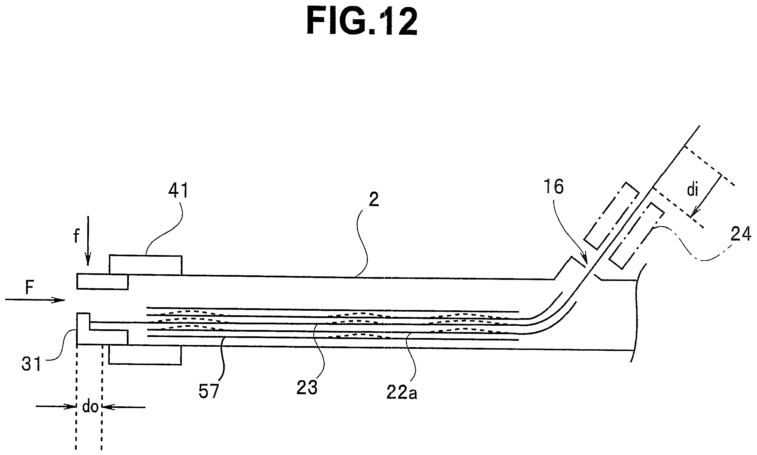

Fig. 12 is a schematic view for describing operations of the endoscope auxiliary instrument. When the proximal end portion of the wire cable 22 is fixed to the wire fixing portion 24 and the surgeon is utilizing the hood 31, the surgeon rotates the handle 84 in the axial direction to release a fixed state of the slider member 83 with respect to the body portion 81, moves the handle 71 to the distal end side, pushes the wire cable 22 into the inside of the opening portion 16 by a predetermined amount, and then rotates the handle 84 once more in a predetermined direction to fix the slider member 83 with respect to the body portion 81.

Because the wire cable 22 has been pushed in toward the distal end side by a predetermined amount, the hood 31 that is connected to the distal end portion of the wire cable 22 protrudes from the distal end portion 2A.

Since a protruding amount d0 of the hood 31 is restricted by the hood cover 42, the protruding amount d0 is not equal to or higher than the predetermined amount. As described above, since the claw portions 31 a of the hood 31 contact against the inner wall on the distal end side of the hole portions 42a of the hood cover 42, the hood 31 does not protrude further forward than the relevant amount.

On the other hand, a pushed-in amount di of the wire cable 22 is set to be greater than the protruding amount d0 of the hood 31 so that, even if the shapes of the bending portion 12 and the insertion portion 2 of the endoscope 1 change, the protruding amount d0 of the hood 31 can be ensured and the wire cable 22 can also generate an elastic force.

When the wire cable 22 is pushed in by the pushed-in amount di, as shown in Fig. 12, the wire cable 22 deforms and enters a curved state inside the channel tube 57. For example, if it is assumed that the length of the insertion portion 2 is 2 meters and the protruding amount d0 of the hood 31 is 3 mm, the pushed-in amount di of the wire cable 22 at the wire fixing portion 24 is taken as 10 mm or more.

In this state, in a case where the proximal end portion of the wire cable 22 is fixed at the wire fixing portion 24, if the hood 31 contacts a mucous membrane or the like inside a body cavity, as indicated by the dotted lines in Fig. 12, the wire cable 22 deforms inside the channel tube 57, and the hood 31 withdraws to the proximal end side under the elastic force of the wire cable 22 itself.

As shown in Fig. 12, when the hood 31 is in a protruding state, although the wire cable 22 is fixed at the wire fixing portion 24, the wire cable 22 is in a taut state while bending inside the channel tube 57, in other words, the wire cable 22 is in a state in which the wire cable 22 is being pressed from the proximal end side. In this state, if an external force F is applied to the hood 31 from the distal end side, the hood 31 withdraws, that is, escapes, in the proximal end direction in accordance with the applied external force. However, when the external force F is no longer applied to the hood 31, the hood 31 returns to the position of the protruded state that is the initial position. That is, since the elastic force of the wire cable 22 itself allows the hood 31 to withdraw with an elastic force with respect to a reactive force when the hood 31 contacts against a body cavity wall, a strong impact is not applied to the body cavity wall.

As described above, the distal end portion of the wire cable 22 is connected to the hood 31. The wire cable 22 is a member that causes the hood 31 to protrude/retract in the distal end portion 2A in parallel with the cylindrical axis of the hood 31 and along the axial direction of the insertion portion 2. Further, the wire cable 22 constitutes a return force generation member that has flexibility and that generates a return force that, when the hood received an external force in the proximal end direction, causes the hood 31 to returns to a protruded state. The wire fixing portion 24 constitutes a fixing portion that fixes the proximal end portion of the wire cable 22. Note that since the hood 31 can maintain a protruded state with respect to the external force F from the radial direction of the hood 31, the function thereof as a hood is maintained when pressing against a wall inside a body cavity or when a lesioned part viewed tangentially is viewed from the front.

Therefore, according to the above described embodiment, an endoscope auxiliary instrument and an endoscope can be realized that can lessen an impact when a hood contacts against living tissue, without increasing the outer diameter of a distal end portion of an insertion portion of the endoscope.

Next, modifications will be described.

### (Modification 1)

A colored ring may be provided in the opening portion 51a to facilitate differentiation between the auxiliary instrument insertion port 51a and an opening portion that is used as an opening portion of the treatment instrument insertion channel when a surgeon inserts the wire cable 22 from the auxiliary instrument insertion port 51a of the auxiliary instrument insertion channel of the endoscope distal end portion 2A.

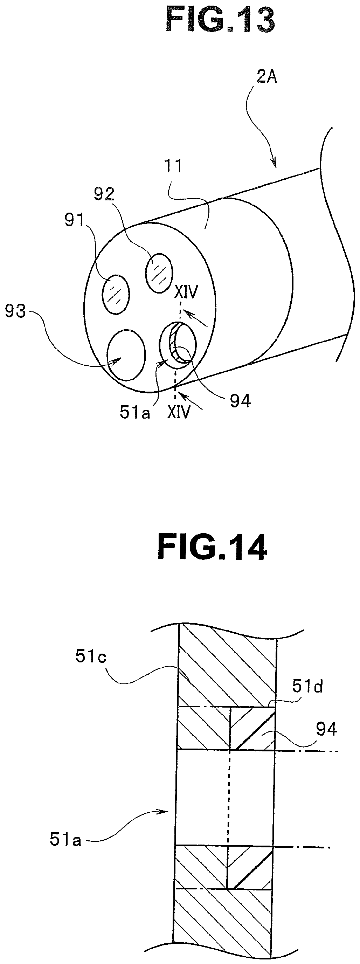

Fig. 13 is a perspective view of the distal end portion 2A in a case where a colored ring is provided in the auxiliary instrument insertion port 51a. In addition to the observation window 91, the illuminating window 92, and the opening portion 93 of the treatment instrument insertion channel, the auxiliary instrument insertion port 51a of the auxiliary instrument insertion channel exists in the rigid member 51. To ensure that the surgeon does not mistakenly insert the wire cable 22 into the opening portion 93 of the treatment instrument insertion channel, a colored ring 94 that is colored with a color that is different to that of members in the surrounding area, such as blue, yellow, or red, is disposed somewhat on the proximal end side of the auxiliary instrument insertion port 51a. The colored ring 94 is disposed at a position that the surgeon can visually recognize when viewing the distal end portion 2A.

Fig. 14 is a partial cross-sectional view of the distal end portion 2A at a region including the colored ring 94 along a line XIV-XIV in Fig. 13. The rigid member 51 has a cover member 51c. The colored ring 94 is attached at the periphery of the auxiliary instrument insertion port 51a on the proximal end side of the cover member 51c. For this purpose, a step portion 51 d is formed at the periphery of the opening portion 51a on the proximal end side of the cover member 51 c, and the colored ring 94 can be provided in the rigid member 51 by fitting the colored ring 94 into the step portion 51d.

By providing the colored ring 94 in this manner, it is possible to prevent a situation in which the surgeon mistakenly inserts the wire cable 22 into the opening portion 93 of the treatment instrument insertion channel.

Note that, although in the example shown in Fig. 13 and Fig. 14 a configuration is adopted in which the step portion 51d is provided on the cover member 51c, and the colored ring 94 is fitted into the step portion 51d, a configuration may also be adopted in which the step portion 51 d is not provided and, as indicated by alternate long and short dash lines in Fig. 14, the colored ring 94 is provided in the cover member 51 c by insert molding or outsert molding.

Note that although in the above described example the colored ring 94 is provided in the auxiliary instrument insertion port 51a of the auxiliary instrument insertion channel, a configuration may also be adopted in which the colored ring 94 is provided in the opening portion 93 of the treatment instrument insertion channel and is not provided in the auxiliary instrument insertion port 51a of the auxiliary instrument insertion channel. When such a configuration is adopted also, since a surgeon can differentiate between the two opening portions, a situation can be prevented in which the surgeon mistakenly inserts the wire cable 22 into the opening portion 93 of the treatment instrument insertion channel.

### (Modification 2)

Similarly to Modification 1, to prevent a situation in which a surgeon mistakenly inserts the wire cable 22 into the opening portion 93 of the treatment instrument insertion channel, a configuration may be adopted in which the auxiliary instrument insertion port 51a of the auxiliary instrument insertion channel is made luminous.

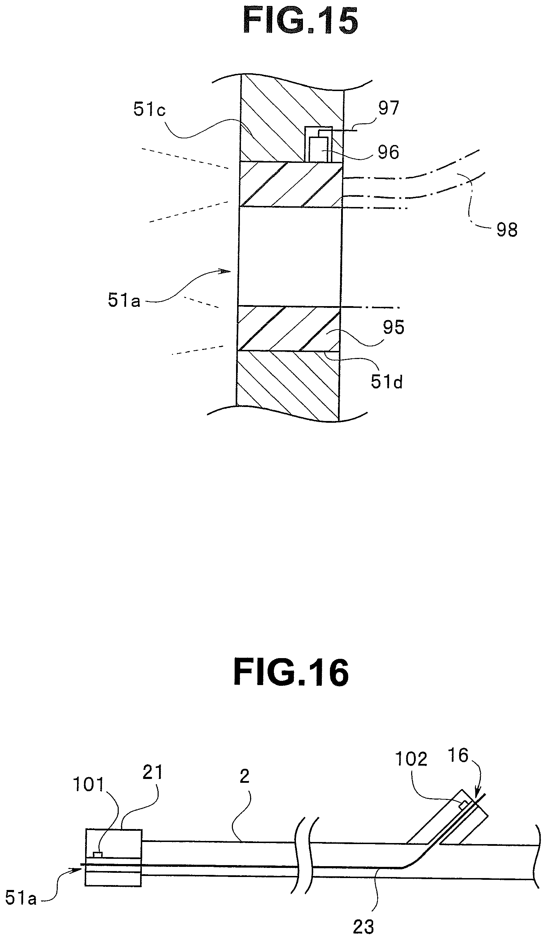

Fig. 15 is a partial cross-sectional view of the distal end portion 2A in which the periphery of the opening portion 51a is made luminous. Fig. 15 is a cross-sectional view of approximately the same position as in Fig. 13.

A ring-shaped member 95 that is made of transparent resin is provided around the periphery of the auxiliary instrument insertion port 51 a, and a light-emitting diode (LED) element (hereunder, referred to as "LED") 96 is provided in close contact with one part on the outer circumferential side of the ring-shaped member 95. When an electric current is supplied to the LED 96 through wiring 97, the LED 96 emits a light, and the light passes through the inside of the ring-shaped member 95 and is emitted from the distal end side.

Hence, according to this configuration also, a situation in which the surgeon mistakenly inserts the wire cable 22 into the opening portion 93 of the treatment instrument insertion channel can be prevented.

Note that, for example, as indicated by alternate long and short dash lines in Fig. 15, if a configuration is adopted in which an LED is not used and a distal end of a light guide 98 from an unshown light source apparatus is disposed in the vicinity of the ring-shaped member 95 and light from the light guide 98 is introduced into the inside of the ring-shaped member 95, since light is emitted from the ring-shaped member 95, a situation in which the surgeon mistakenly inserts the wire cable 22 into the opening portion 93 of the treatment instrument insertion channel can be prevented.

Furthermore, a similar effect is obtained when a material including a fluorescent substance having a phosphorescent property is used for the ring-shaped member 95.

### (Modification 3)

Similarly to Modifications 1 and 2, to prevent a situation in which the surgeon mistakenly inserts the wire cable 22 into the opening portion 93 of the treatment instrument insertion channel, a configuration may be adopted in which a sensor is provided inside the auxiliary instrument insertion channel to detect insertion of the wire cable 22 into the auxiliary instrument insertion port 51a.

Fig. 16 is a view for describing the configuration of Modification 3. As shown in Fig. 16, a sensor 101 that senses insertion of the wire cable 22 into the auxiliary instrument insertion channel is provided in the vicinity of the auxiliary instrument insertion port 51a. The output of the sensor 101 is supplied to an unshown control portion (for example, a processor of an endoscope apparatus), so that detection by the sensor 101 of the wire cable 22 when the wire cable 22 is inserted from the endoscope distal end portion 2A is notified to the surgeon by means of a display on a monitor or by sounding of a buzzer or the like.

The sensor 101 is, for example, a photocoupler or a color sensor. When a photocoupler is used, it is possible to detect that light has been interrupted by the wire cable 22. When a color sensor is used, the color of the wire cable 22 can be detected.

Since it is thus possible for a surgeon to know when the wire cable 22 has been correctly inserted into the auxiliary instrument insertion port 51a, a situation in which the surgeon mistakenly inserts the wire cable 22 into the opening portion 93 of the treatment instrument insertion channel can be prevented.

Note that a configuration may also be adopted in which a sensor 102 is provided in the vicinity of the opening portion 16.

### (Modification 4)

Although in each of above described examples an insertion channel for an endoscope auxiliary instrument is utilized to pass through the wire cable 22 of the endoscope auxiliary instrument, a configuration may also be adopted in which the treatment instrument insertion channel of the endoscope is utilized, and an insertion channel for an endoscope auxiliary instrument is not provided in the endoscope.

### (Modification 5)

Although in each of above described examples the insertion channel for an endoscope auxiliary instrument for passing through the wire cable 22 of the endoscope auxiliary instrument is provided inside the endoscope insertion portion 2, a configuration may also be adopted in which the insertion channel for the endoscope auxiliary instrument is provided outside the endoscope insertion portion 2. For example, a configuration may be adopted in which a tube for passing through the wire cable 22 is provided on the outer circumference of the insertion portion 2, and a conduit of the tube is utilized as an insertion channel for the endoscope auxiliary instrument.

### (Modification 6)

In each of above described examples the hood 31 is provided inside the unit body 41 that is mounted to the distal end portion of the endoscope insertion portion. However, a configuration may also be adopted in which the hood 31 is provided inside the distal end rigid portion 11 of the distal end portion of the endoscope insertion portion. That is, the hood 31 may be configured in an integrated manner with respect to the distal end rigid portion 11.

In this case, the hood cover 42 is provided in the distal end rigid portion 11 or, alternatively, a structure having similar functions as the above described hood cover 42 is provided in the distal end rigid portion 11. By adopting this configuration, there is the advantage that work to mount the hood unit is not required.

### (Modification 7)

A configuration may also be adopted in which the hood 31 is provided on the outer circumference of the distal end rigid portion 11 of the endoscope insertion portion. In this case, a convex portion and a concave portion that engage with each other are provided on the inner circumference of the hood 31 and the outer circumference of the distal end rigid portion 11 so that the hood 31 is capable of advancing and retracting in the longitudinal axis direction with respect to the distal end rigid portion 11. By adopting this configuration, there are the advantages that the hood cover 42 is not required, and it is not necessary to decrease the outer diameter of the distal end when the hood is mounted or to perform work to mount the hood unit.

As described above, according to the foregoing embodiment and the respective modifications, an endoscope auxiliary instrument and an endoscope can be provided that can lessen an impact when a hood contacts against living tissue, without increasing the outer diameter of a distal end portion of the endoscope and without lengthening a distal end rigid portion.

The present invention is not limited to the above described embodiment, and various changes and alterations can be made within a range that does not depart from the scope of the present invention.

## Claims

1. An endoscope auxiliary instrument, comprising:
a cylindrical member (31), wherein the endoscope auxiliary instrument further comprises:
a holding portion (42) that is provided at a distal end portion of an insertion portion of an endoscope, and that is adapted to movably hold the cylindrical member along an axial direction of the insertion portion, the holding portion having a protruding portion that protrudes more in a distal end direction of a distal end portion of the insertion portion than a distal end face of the distal end portion of the insertion portion of the endoscope;
a return force generation member (22) that has a distal end portion and a proximal end portion, in which the distal end portion is fixed to the cylindrical member, and that is adapted to cause the cylindrical member to protrude and retract at the distal end portion in a direction that is parallel to an axis of the cylindrical member and is along the axial direction of the insertion portion, and when the cylindrical member receives a force in a proximal end direction in a state in which the cylindrical member protrudes in a distal end direction of the distal end portion, is adapted to generate a return force that causes the cylindrical member to return to the protruding state; and
a fixing portion (24) that is fixed to an opening portion in an insertion channel provided inside the insertion portion and extending from an insertion port provided in the distal end face of the distal end portion of the insertion portion and comprising the opening portion on a proximal end side of the insertion portion, and that is capable of switching the return force generation member that is inserted into the insertion port from a proximal end portion of the return force generation member and passes through the insertion channel and is protruded from the opening portion between a state in which the return force generation member is fixed to the fixing portion and a state in which the return force generation member is not fixed thereto.

2. The endoscope auxiliary instrument according to claim 1, wherein:
the holding portion comprises a body portion and a cover member; and
the protruding portion is provided in the cover member.

3. The endoscope auxiliary instrument according to claim 1, wherein:
the holding portion comprises a body portion and a cover member;
the cover member has a cylindrical shape in which a tapered portion is formed along an outer circumference of a distal end portion;
the cylindrical member has a curved portion along an outer circumference of a distal end portion; and
when the cylindrical member withdraws to a proximal end side of the distal end portion, an inclining line of the tapered portion of the cover member and a tangent line of the curved portion of the cylindrical member approximately coincide with each other.

4. The endoscope auxiliary instrument according to claim 1, wherein:
the return force generation member is a wire that has flexibility; and
a distal end portion of the wire is connected to the cylindrical member.

5. The endoscope auxiliary instrument according to claim 4, wherein
the insertion channel has a tube that is connected to a rigid member that is provided at a distal end portion of the insertion portion of the endoscope; and
the wire is inserted through an inside of the tube.

6. The endoscope auxiliary instrument according to claim 1, wherein the holding portion has a movement restricting portion that restricts movement of the cylindrical member along the axial direction of the insertion portion.

7. The endoscope auxiliary instrument according to claim 6, wherein:
the cylindrical member has a claw portion; and
movement of the cylindrical member along the axial direction of the insertion portion is restricted by contact of the claw portion against the movement restricting portion.

8. The endoscope auxiliary instrument according to claim 7, wherein:
the holding portion comprises a body portion and a cover member; and
the movement restricting portion is provided in the cover member.

9. An endoscope comprising the endoscope auxiliary instrument according to claim 1.

## Patentansprüche

1. Endoskophilfsinstrument, das umfasst:
ein zylindrisches Element (31), wobei das Endoskophilfsinstrument ferner umfasst:
einen Halteabschnitt (42), der an einem distalen Endabschnitt eines Einführabschnitts eines Endoskops vorgesehen und eingerichtet ist, das zylindrische Element entlang einer axialen Richtung des Einführabschnitts bewegbar zu halten, wobei der Halteabschnitt einen vorstehenden Abschnitt aufweist, der mehr in eine distale Endrichtung eines distalen Endabschnitts des Einführabschnitts vorsteht als eine distale Endfläche des distalen Endabschnitts des Einführabschnitts des Endoskops;
ein Rückstellkrafterzeugungselement (22), das einen distalen Endabschnitt und einen proximalen Endabschnitt besitzt, in dem der distale Endabschnitt an dem zylindrischen Element befestigt ist, und das eingerichtet ist, das zylindrische Element zu veranlassen, sich an dem distalen Endabschnitt in eine Richtung vorzuschieben und zurückzuziehen, die parallel zu einer Achse des zylindrischen Elements und entlang der axialen Richtung des Einführabschnitts ist, und eingerichtet ist, eine Rückstellkraft zu erzeugen, die das zylindrische Element veranlasst, in den vorstehenden Zustand zurückzukehren, wenn das zylindrische Element in einem Zustand, in dem das zylindrische Element in eine distale Endrichtung des distalen Endabschnitts vorsteht, eine Kraft in eine proximale Endrichtung aufnimmt; und
einen Befestigungsabschnitt (24), der an einem Öffnungsabschnitt in einem Einführkanal, der in dem Einführabschnitt vorgesehen ist und sich von einem Einführzugang, der in der distalen Endfläche des distalen Endabschnitts des Einführabschnitts vorgesehen ist und den Öffnungsabschnitt an einer proximalen Endseite des Einführabschnitts umfasst, erstreckt, befestigt und fähig ist, das Rückstellkrafterzeugungselement, das von einem proximalen Endabschnitt des Rückstellkrafterzeugungselements in den Einführzugang eingeführt ist und durch den Einführkanal läuft und aus dem Öffnungsabschnitt vorsteht, zwischen einem Zustand, in dem das Rückstellkrafterzeugungselement an dem Befestigungsabschnitt befestigt ist, und einem Zustand, in dem das Rückstellkrafterzeugungselement nicht daran befestigt ist, umzuschalten.

2. Endoskophilfsinstrument nach Anspruch 1, wobei:
der Halteabschnitt einen Körperabschnitt und ein Abdeckelement umfasst; und
der vorstehende Abschnitt in dem Abdeckelement vorgesehen ist.

3. Endoskophilfsinstrument nach Anspruch 1, wobei:
der Halteabschnitt einen Körperabschnitt und ein Abdeckelement umfasst;
das Abdeckelement eine zylindrische Form hat, in dem entlang eines äußeren Umfangs eines distalen Endabschnitts ein zulaufender Abschnitt geformt ist;
das zylindrische Element entlang eines äußeren Umfangs eines distalen Endabschnitts einen gekrümmten Abschnitt aufweist; und
eine geneigte Linie des zulaufenden Abschnitts des Abdeckelements und eine Tangentenlinie des gekrümmten Abschnitts des zylindrischen Elements ungefähr übereinander liegen, wenn sich das zylindrische Element zu einer proximalen Endseite des distalen Endabschnitts zurückzieht.

4. Endoskophilfsinstrument nach Anspruch 1, wobei:
das Rückstellkrafterzeugungselement ein Draht ist, der Flexibilität aufweist; und
ein distaler Endabschnitt des Drahts mit dem zylindrischen Element verbunden ist.

5. Endoskophilfsinstrument nach Anspruch 4, wobei
der Einführkanal eine Röhre besitzt, die mit einem steifen Element, das an einem distalen Endabschnitt des Einführabschnitts des Endoskops vorgesehen ist, verbunden ist; und
der Draht durch ein Inneres der Röhre eingeführt ist.

6. Endoskophilfsinstrument nach Anspruch 1, wobei der Halteabschnitt einen bewegungsbeschränkenden Abschnitt aufweist, der eine Bewegung des zylindrischen Elements entlang der axialen Richtung des Einführabschnitts beschränkt.

7. Endoskophilfsinstrument nach Anspruch 6, wobei:
das zylindrische Element einen Krallenabschnitt aufweist; und
eine Bewegung des zylindrischen Elements entlang der axialen Richtung des Einführabschnitts durch Kontakt des Krallenabschnitt gegen den bewegungsbeschränkenden Abschnitt beschränkt wird.

8. Endoskophilfsinstrument nach Anspruch 7, wobei:
der Halteabschnitts einen Körperabschnitt und ein Abdeckelement umfasst; und
der bewegungsbeschränkende Abschnitt in dem Abdeckelement vorgesehen ist.

9. Endoskop, das das Endoskophilfsinstrument nach Anspruch 1 umfasst.

## Revendications

1. Instrument endoscopique auxiliaire, comprenant :
un élément cylindrique (31) dans lequel l'instrument endoscopique auxiliaire comprend en outre :
une partie de maintien (42) qui est prévue au niveau d'une partie d'extrémité distale d'une partie d'insertion d'un endoscope, et qui est adaptée pour maintenir de manière mobile l'élément cylindrique le long d'une direction axiale de la partie d'insertion, la partie de maintien comportant une partie de saillie qui fait saillie davantage dans la direction d'extrémité distale d'une partie d'extrémité distale de la partie d'insertion qu'une face d'extrémité distale de la partie d'extrémité distale de la partie d'insertion de l'endoscope ;
un élément (22) de génération de force de retour qui comporte une partie d'extrémité distale et une partie d'extrémité proximale, dans lequel la partie d'extrémité distale est fixée à l'élément cylindrique, et qui est adapté pour amener l'élément cylindrique à faire saillie et à se rétracter au niveau de la partie d'extrémité distale dans une direction qui est parallèle à un axe de l'élément cylindrique et se situe le long de la direction axiale de la partie d'insertion, et lorsque l'élément cylindrique reçoit une force dans une direction d'extrémité proximale dans un état dans lequel l'élément cylindrique fait saillie dans une direction d'extrémité distale de la partie d'extrémité distale, est adapté pour générer une force de retour qui amène l'élément cylindrique à retourner à l'état de saillie ; et
une partie de fixation (24) qui est fixée à une partie d'ouverture dans un canal d'insertion prévu à l'intérieur de la partie d'insertion et s'étendant à partir d'un orifice d'insertion prévu dans la face d'extrémité distale de la partie d'extrémité distale de la partie d'insertion et comprenant la partie d'ouverture sur un côté d'extrémité proximale de la partie d'insertion, et qui est capable de commuter l'élément de génération de force de retour qui est inséré dans l'orifice d'insertion d'une partie d'extrémité proximale de l'élément de génération de force de retour et passe à travers le canal d'insertion et fait saillie depuis la partie d'ouverture entre un état dans lequel l'élément de génération de force de retour est fixé à la partie de fixation et un état dans lequel l'élément de génération de force de retour n'est pas fixé à celle-ci.

2. Instrument endoscopique auxiliaire selon la revendication 1, dans lequel :
la partie de maintien comprend une partie de corps et un élément de couvercle ; et
la partie de saillie est prévue dans l'élément de couvercle.

3. Instrument endoscopique auxiliaire selon la revendication 1, dans lequel :
la partie de maintien comprend une partie de corps et un élément de couvercle ;
l'élément de couvercle présente une forme cylindrique dans laquelle une partie conique est formée le long d'une circonférence externe d'une partie d'extrémité distale ;
l'élément cylindrique comporte une partie incurvée le long d'une circonférence externe d'une partie d'extrémité distale ; et
lorsque l'élément cylindrique se retire vers un côté d'extrémité proximale de la partie d'extrémité distale, une ligne d'inclinaison de la partie conique de l'élément de couvercle et une ligne tangente de la partie incurvée de l'élément cylindrique coïncident approximativement l'une avec l'autre.

4. Instrument endoscopique auxiliaire selon la revendication 1, dans lequel :
l'élément de génération de force de retour est un câble qui possède la flexibilité ; et
une partie d'extrémité distale du câble est reliée à l'élément cylindrique.

5. Instrument endoscopique auxiliaire selon la revendication 4, dans lequel :
le canal d'insertion comporte un tube qui est raccordé à un élément rigide qui est prévu au niveau d'une partie d'extrémité distale de la partie d'insertion de l'endoscope ; et
le câble est inséré à travers l'intérieur du tube.

6. Instrument endoscopique auxiliaire selon la revendication 1, dans lequel la partie de maintien comporte une partie de limitation de mouvement qui limite le mouvement de l'élément cylindrique le long de la direction axiale de la partie d'insertion.

7. Instrument endoscopique auxiliaire selon la revendication 6, dans lequel :
l'élément cylindrique comporte une partie de griffe ; et
le mouvement de l'élément cylindrique le long de la direction axiale de la partie d'insertion est limité par contact de la partie de griffe contre la partie de limitation de mouvement.

8. Instrument endoscopique auxiliaire selon la revendication 7, dans lequel :
la partie de maintien comprend une partie de corps et un élément de couvercle ; et
la partie de limitation de mouvement est prévue dans l'élément de couvercle.

9. Endoscope comprenant l'instrument endoscopique auxiliaire selon la revendication 1.
